# EUROPÄISCHE PATENTSCHRIFT

(11) **EP 1 215 210 B1**
(45) Veröffentlichungstag und Bekanntmachung des Hinweises auf die Patenterteilung: **18.10.2006**
(21) Anmeldenummer: 02006144.6
(22) Anmeldetag: 11.04.1996
(51) Int. Cl.: C07D 307/85, C07D 405/12, A61K 31/50, A61K 31/34

(54) **Benzofurane**
Benzofurans
Benzofurannes

(30) Priorität: 20.04.1995 DE 19514567
(43) Veröffentlichungstag der Anmeldung: 19.06.2002
(62) Teilanmeldung aus: 96105701.5
(73) Patentinhaber: Merck Patent GmbH, 64293 Darmstadt (DE)
(72) Erfinder: Bathe, Andreas, Dr., 64283 Darmstadt (DE); Helfert, Bernd, Dr., 64373 Ober-Ramstadt (DE); Böttcher, Henning, Dr., 64287 Darmstadt (DE); Schuster, Kurt, Dr., 64372 Darmstadt (DE)

(56) Entgegenhaltungen:
- DE-A- 4 333 254
- J. KAHOVCOVÁ ET A.: "Natural and synthetic materials with insect hormone activity. XVI. The synthesis of derivatives of oxygen heterocycles containing N-geranylaniline moiety" COLLECTION OF CZECHOSLOVAK CHEMICAL COMMUNICATIONS, Bd. 38, Nr. 4, 1973, Seiten 1165-1167, XP002009856 PRAGUE CS
- H. ERLENMEYER ET AL.: "Zur Kenntnis des 5-Aminocumarons" HELVETICA CHIMICA ACTA, Bd. 31, 1948, Seiten 75-77, XP002009857 BASEL CH

## Beschreibung

Die Erfindung betrifft Benzofurane der Formel I 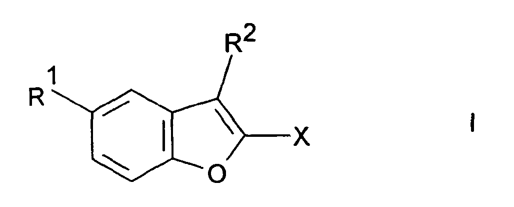 worin
- R¹: NH₂ oder 1-Piperazinyl
- R²: H, Cl, Br, OH oder OA,
- X: CONR⁴R⁵ oder CO-Het,
- R⁴ und R⁵: jeweils unabhängig voneinander H, A oder Benzyl,
- A: Alkyl mit 1-4 C-Atomen,
- Het: Imidazol-1-yl, Triazol-1-yl oder Tetrazol-1-yl
bedeuten,
sowie deren Salze.

Ähnliche Verbindungen sind aus DE 43 33 254 bekannt.

Der Erfindung lag die Aufgabe zugrunde, neue Verbindungen zu finden, die insbesondere als Zwischenprodukte bei der Synthese von Arzneimitteln verwendet werden können, aber auch direkt zur Herstellung von Arzneimitteln Verwendung finden können.

Es wurde gefunden, daß die Verbindungen der Formel I und ihre Salze wichtige Zwischenprodukte zur Herstellung von Arzneimitteln darstellen und zugleich pharmakologische Eigenschaften besitzen. So zeigen sie beispielsweise Wirkungen auf das Zentralnervensystem.

Gegenstand der Erfindung sind die Benzofuranderivate der Formel I und ihre Salze.

Vor- und nachstehend haben die Reste R¹, R², R³, R⁴, R⁵, R⁶, X, X¹, X², A, und Het die bei den Formeln I bis VI angegebenen Bedeutungen, falls nicht ausdrücklich etwas anderes angegeben ist.

In den vorstehenden Formeln hat A 1 bis 4, vorzugsweise 1, 2, oder 3 C-Atome. A bedeutet vorzugsweise Methyl oder Ethyl, weiterhin Propyl, Isopropyl, ferner auch Butyl, Isobutyl, sek.-Butyl oder tert.-Butyl.

Der Rest Het bedeutet ein in der 1-Position substituiertes Imidazol, Triazol oder Tetrazol.

Der Rest X bedeutet CONR⁴R⁵ oder CO-Het.

Der Rest R¹ bedeutet NH₂ oder 1-Piperazinyl

Der Rest R² bedeutet H, Cl, Br, OH oder OA.

Der Rest R³ bedeutet Benzyl, vorzugsweise aber eine an sich bekannte Aminoschutzgruppe.

Der Ausdruck "Aminoschutzgruppe" ist allgemein bekannt und bezieht sich auf Gruppen, die geeignet sind, eine Aminogruppe vor chemischen Umsetzungen zu schützen (zu blockieren), die aber leicht entfernbar sind, nachdem die gewünschte chemische Reaktion an anderen Stellen des Moleküls durchgeführt worden ist. Typisch für solche Gruppen sind insbesondere unsubstituierte Acyl-, Aryl-, Aralkoxymethyl- oder Aralkylgruppen. Da die Aminoschutzgruppen nach der gewünschten Reaktion (oder Reaktionsfolge) entfernt werden, ist ihre Art und Größe im übrigen nicht kritisch; bevorzugt werden jedoch solche mit 1-20, insbesondere 1-8 C-Atomen. Der Ausdruck "Acylgruppe" ist im Zusammenhang mit dem vorliegenden Verfahren und den vorliegenden Verbindungen in weitestem Sinne aufzufassen. Er umschließt von aliphatischen, araliphatischen, aromatischen oder heterocyclischen Carbonsäuren oder Sulfonsäuren abgeleitete Acylgruppen sowie insbesondere Alkoxycarbonyl-, Aryloxycarbonyl- und vor allem Aralkoxycarbonylgruppen. Beispiele für derartige Acylgruppen sind Alkanoyl wie Acetyl, Propionyl, Butyryl; Aralkanoyl wie Phenylacetyl; Aroyl wie Benzoyl oder Toluyl; Aryloxyalkanoyl wie Phenoxyacetyl; Alkoxycarbonyl wie Methoxycarbonyl, Ethoxycarbonyl, 2,2,2-Trichlorethoxycarbonyl, BOC (tert.-Butoxycarbonyl), 2-lodethoxycarbonyl; Aralkyloxycarbonyl wie CBZ (Carbobenzoxycarbonyl, auch "Z" genannt), 4-Methoxybenzyloxycarbonyl, FMOC (9-Fluorenylmethoxycarbonyl); Arylsulfonyl wie Mtr (4-Methoxy-2,3,6-trimethylphenyl-sulfonyl). Bevorzugte Aminoschutzgruppen sind BOC und Mtr, ferner CBZ oder FMOC.

Die Verbindungen der Formel I können ein oder mehrere chirale Zentren besitzen und daher in verschiedenen stereoisomeren Formen vorkommen. Die Formel I umschließt alle diese Formen.

Gegenstand der Erfindung ist ferner ein Verfahren zur Herstellung von Benzofuranen der Formel I sowie von deren Salze, dadurch gekennzeichnet, daß man eine Verbindung, die der Formel I entspricht, worin statt R¹ eine Nitrogruppe steht,
auf übliche Weise reduziert,
oder
daß man eine Verbindung, die der Formel I entspricht,
worin R¹ eine NH₂-Gruppe ist,
mit einer Verbindung der Formel II

R⁶N(CH₂CH₂X¹)₂ II

worin
- R⁶: H oder Benzyl und
- X¹: Cl, Br, I, OH oder eine reaktionsfähig funktionell abgewandelte OH-Gruppe bedeutet,
umsetzt,
oder
daß man eine Verbindung, die der Formel I entspricht, worin statt X COOA-Gruppe steht, worin A die angegenene Bedeutung besitzt,
in eine andere Verbindung der Formel I umwandelt, in der X CONR⁴R⁵ bedeutet, worin R⁴ und R⁵ die angegebenen Bedeutungen besitzen,
oder
daß man eine Verbindung, die der Formel I entspricht, worin statt X eine COOH-Gruppe steht,
in eine andere Verbindung der Formel I umwandelt, in der X CO-Het bedeutet, worin Het die angegebene Bedeutung besitzt,
oder
daß man eine Verbindung, die der Formel I entspricht,
worin statt R¹ eine 4-R³-Piperazinylgruppe steht, worin R³ die angegebene Bedeutung besitzt,
durch Abspaltung der Schutzgruppe in eine Verbindung der Formel I umwandelt, in der R¹ 1-Piperazinyl bedeutet
und/oder
daß man eine Base der Formel I durch Behandeln mit einer Säure in eines ihrer Salze umwandelt.

Die Verbindungen der Formel I und auch die Ausgangsstoffe zu ihrer Herstellung werden im übrigen nach an sich bekannten Methoden hergestellt, wie sie in der Literatur (z.B. in den Standardwerken wie Houben-Weyl, Methoden der organischen Chemie, Georg-Thieme-Verlag, Stuttgart) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können, falls erwünscht, auch in situ gebildet werden, so daß man sie aus dem Reaktionsgemisch nicht isoliert, sondern sofort weiter zu den Verbindungen der Formel I umsetzt.

In den Verbindungen der Formel II bedeutet der Rest X¹ vorzugsweise Cl oder Br; er kann jedoch auch I, OH oder eine reaktionsfähig abgewandelte OH-Gruppe wie Alkylsulfonyloxy mit 1-6 C-Atomen (bevorzugt Methylsulfonyloxy) oder Arylsulfonyloxy mit 6-10 C-Atomen (bevorzugt Phenyl-, p-Tolylsulfonyloxy, 1- oder 2-Naphthalinsulfonyloxy) bedeuten.

In den Verbindungen der Formel II bedeutet der Rest R⁶ H oder Benzyl. Die Verbindungen der Formel II sind zum Teil bekannt; die nicht bekannten Verbindungen können leicht analog zu den bekannten Verbindungen hergestellt werden.

Die Umsetzung der Verbindungen der Formel II mit Verbindungen der Formel I, in denen R¹ NH₂ bedeutet verläuft nach Methoden, wie sie für die Alkylierung von Aminen aus der Literatur bekannt sind. Man kann ohne Gegenwart eines Lösungsmittels die Komponenten miteinander verschmelzen, gegebenenfalls im geschlossenen Rohr oder im Autoklaven.

Es ist aber auch möglich, die Verbindungen in Gegenwart eines inerten Lösungsmittels umzusetzen.

Als inerte Lösungsmittel eignen sich z.B. Kohlenwasserstoffe wie Hexan, Petrolether, Benzol, Toluol oder Xylol; chlorierte Kohlenwassertoffe wie Trichlorethylen, 1,2-Dichlorethan, Tetrachlorkohlenstoff, Chloroform oder Dichlormethan; Alkohole wie Methanol, Ethanol, Isopropanol, n-Propanol, n-Butanol oder tert.-Butanol; Ether wie Diethylether, Diisopropylether, Tetrahydrofuran (THF) oder Dioxan; Glykolether wie Ethylenglykolmonomethyl- oder -monoethylether (Methylglykol oder Ethylglykol), Ethylenglykoldimethylether (Diglyme); Ketone wie Aceton oder Butanon; Amide wie Acetamid, Dimethylacetamid oder Dimethylformamid (DMF); Nitrile wie Acetonitril; Sulfoxide wie Dimethylsulfoxid (DMSO); Schwefelkohlenstoff; Nitroverbindungen wie Nitromethan oder Nitrobenzol; Ester wie Ethylacetat, gegebenenfalls auch Gemische der genannten Lösungsmittel untereinander oder Gemische mit Wasser.

Der Zusatz eines säurebindenden Mittels, beispielsweise eines Alkali- oder Erdalkalimetall-hydroxids, -carbonats oder -bicarbonats oder eines anderen Salzes einer schwachen Säure der Alkali- oder Erdalkalimetalle, vorzugsweise des Kaliums, Natriums oder Calciums, oder der Zusatz einer organischen Base wie Triethylamin, Dimethylamin, Pyridin oder Chinolin oder eines Überschusses der Aminkomponente kann günstig sein. Die Reaktionszeit liegt je nach den angewendeten Bedingungen zwischen einigen Minuten und 14 Tagen, die Reaktionstemperatur zwischen 0 und 150°, normalerweise zwischen 20 und 130°.

Die Umwandlung einer Verbindung der Formel I, in der statt R¹ eine Nitrogruppe steht, in eine Verbindung der Formel I, in der R¹ eine Aminogruppe bedeutet, erfolgt vorzugsweise mit Wasserstoffgas unter Übergangsmetallkatalyse (beispielsweise durch Hydrierung an Raney-Nickel oder Pd-Kohle in einem inerten Lösungsmittel wie Methanol oder Ethanol).

Die Umwandlung einer Verbindung der Formel I, worin X eine Carboxygruppe ist, in eine Verbindung der Formel I, in der X CO-Het bedeutet, erfolgt nach bekannten Methoden, wie sie in der Literatur (z.B. in den Standardwerken wie Houben-Weyl, Methoden der organischen Chemie, Georg-Thieme-Verlag, Stuttgart;) für derartige Veresterungen oder Amidierungen beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Umwandlung von Verbindungen der Formel I, in der statt X COOA, COOPh, COOCH₂Ph oder CO-Het bedeutet, in Verbindungen der Formel I, in der X CONR⁴R⁵ bedeutet, erfolgt beispielsweise mit HCONR⁴R⁵ in einem, wie oben angegebenen, inerten Lösungsmittel, gegebenenfalls unter Zusatz einer Base. Als Base dienen z.B. ein Kalium- oder Natriumalkoholat wie Kalium- oder Natriummethylat, -ethylat oder - tert.-butylat.

Die Abspaltung eines Restes R³ erfolgt - je nach der benutzten Schutzgruppe - z.B. mit starken Säuren, zweckmäßig mit TFA (Trifluoressigsäure) oder Perchlorsäure, aber auch mit anderen starken anorganischen Säuren wie Salzsäure oder Schwefelsäure, starken organischen Carbonsäuren wie Trichloressigsäure oder Sulfonsäuren wie Benzol- oder p-Toluolsulfonsäure. Die Anwesenheit eines zusätzlichen inerten Lösungsmittels ist möglich, aber nicht immer erforderlich. Als inerte Lösungsmittel eignen sich vorzugsweise organische, beispielsweise Carbonsäuren wie Essigsäure, Ether wie Tetrahydrofuran oder Dioxan, Amide wie Dimethylformamid, halogenierte Kohlenwasserstoffe wie Dichlormethan, ferner auch Alkohole wie Methanol, Ethanol oder Isopropanol sowie Wasser. Ferner kommen Gemische der vorgenannten Lösungsmittel in Frage. TFA wird vorzugsweise im Überschuß ohne Zusatz eines weiteren Lösungsmittels verwendet, Perchlorsäure in Form eines Gemisches aus Essigsäure und 70 %iger Perchlorsäure im Verhältnis 9 : 1. Die Reaktionstemperaturen liegen zweckmäßig zwischen etwa 0 und etwa 50°, vorzugsweise arbeitet man zwischen 15 und 30°.

Die Gruppe BOC wird bevorzugt mit TFA in Dichlormethan oder mit etwa 3 bis 5 n Salzsäure in Dioxan bei 15-30° abgespalten.

Hydrogenolytisch entfernbare Schutzgruppen (z.B. CBZ oder Benzyl) können z.B. durch Behandeln mit Wasserstoff in Gegenwart eines Katalysators (z.B. eines Edelmetallkatalysators wie Palladium, zweckmäßig auf einem Träger wie Kohle) abgespalten werden. Als Lösungsmittel eignen sich dabei die oben angegebenen, insbesondere z.B. Alkohole wie Methanol oder Ethanol oder Amide wie DMF. Die Hydrogenolyse wird in der Regel bei Temperaturen zwischen etwa 0 und 100° und Drucken zwischen etwa 1 und 200 bar, bevorzugt bei 20-30° und 1-10 bar durchgeführt.

Eine Base der Formel I kann mit einer Säure in das zugehörige Säureadditionssalz übergeführt werden, beispielsweise durch Umsetzung äquivalenter Mengen der Base und der Säure in einem inerten Lösungsmittel wie Ethanol und anschließendes Eindampfen. Für diese Umsetzung kommen insbesondere Säuren in Frage, die physiologisch unbedenkliche Salze liefern. So können anorganische Säuren verwendet werden, z.B. Schwefelsäure, Salpetersäure, Halogenwasserstoffsäuren wie Chlorwasserstoffsäure oder Bromwasserstoffsäure, Phosphorsäuren wie Orthophosphorsäure, Sulfaminsäure, ferner organische Säuren, insbesondere aliphatische, alicyclische, araliphatische, aromatische oder heterocyclische ein- oder mehrbasige Carbon-, Sulfon- oder Schwefelsäuren, z.B. Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Naphthalin-mono- und Disulfonsäuren, Laurylschwefelsäure. Salze mit physiologisch nicht unbedenklichen Säuren, z.B. Pikrate, können zur Isolierung und /oder Aufreinigung der Verbindungen der Formel I verwendet werden.

Andererseits können Verbindungen der Formel I mit Basen (z.B. Natrium- oder Kaliumhydroxid oder -carbonat) in die entsprechenden Metall-, insbesondere Alkalimetall- oder Erdalkalimetall-, oder in die entsprechenden Ammoniumsalze umgewandelt werden.

Gegenstand der Erfindung sind auch Arzneimittel der Formel I und ihre physiologisch unbedenklichen Salze.

Gegenstand der Erfindung ist weiterhin die Verwendung der Verbindungen der Formel I als Zwischenprodukte zur Synthese von Arzneimitteln. Entsprechende Arzneimittel sind beispielsweise in DE 4333254 beschrieben.

Gegenstand der Erfindung ist dementsprechend insbesondere die Verwendung der Verbindungen der Formel I nach Anspruch 1 bei der Synthese von
1-[4-(5-Cyan-indol-3-yl)-butyl]-4-(2-carbamoyl-benzofuran-5-yl)-piperazin
und dessen Salzen, dadurch gekennzeichnet,
daß man 2-(2-Formylphenoxy)-essigsäure nitriert,
daß man die so erhaltene 2-(2-Formyl-4-nitrophenoxy)-essigsäure cyclisiert,
daß man die so erhaltene 5-Nitrobenzofuran-2-carbonsäure verestert,
daß man die so erhaltene Verbindung der Formel III 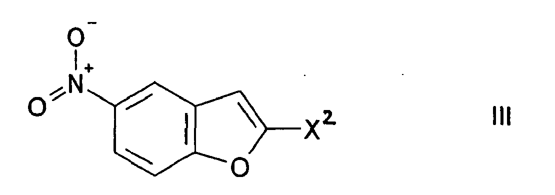 worin X² COOA, COOPh oder COOCH₂Ph
bedeutet,
reduziert,
daß man die so erhaltene Verbindung der Formel I,
worin R¹ NH₂ und anstelle von X eine gruppe COOA, COOPh oder COOCH₂Ph steht,
in ihr Säureadditionssalz umwandelt und dieses mit einem Säureadditionssalz einer Verbindung der Formel II

HN(CH₂CH₂X¹)₂ II

worin X¹ Cl, Br, I, OH oder eine reaktionsfähig funktionell abgewandelte OH-Gruppe bedeutet,
zu einem Piperazinderivat der Formel IV 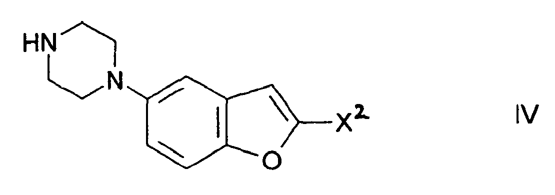 worin X² COOA, COOPh oder COOCH₂Ph bedeutet
umsetzt,
daß man die so erhaltene Verbindung der Formel IV
in ihr Säureadditionssalz umwandelt,
daß man dieses mit einer Verbindung umsetzt, die zur Einführung einer Aminoschutzgruppe geeignet ist,
daß man die so erhaltene Verbindung der Formel V 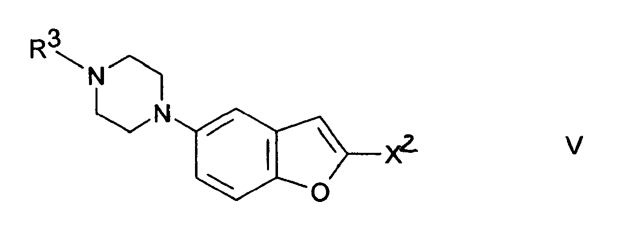 worin R³ eine an sich bekannte Aminoschutzgruppe bedeutet und X² die angegebene Bedeutung besitzt,
in eine Verbindung der Formel VI 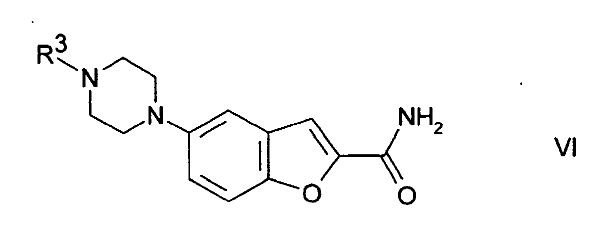 worin R³ die obige Bedeutung hat,
umwandelt,
daß man die so erhaltene Verbindung der Formel VI durch Abspaltung der Aminoschutzgruppe in 5-(1-Piperazinyl)-benzofuran-2-carboxamid oder ein Säureadditionssalz umwandelt und
daß man 5-(1-Piperazinyl)-benzofuran-2-carboxamid oder ein entsprechendes Salz mit 3-(4-Chlorbutyl)-5-cyan-indol zu 1-[4-(5-Cyan-indol-3-yl)-butyl]-4-(2-carbamoyl-benzofuran-5-yl)-piperazin umsetzt und gegebenenfalls anschließend in ein Säureadditionssalz überführt.

3-(4-Chlorbutyl)-5-cyan-indol ist bekannt aus DE 4101686, 1-[4-(5-Cyan-indol-3-yl)-butyl]-4-(2-carbamoyl-benzofuran-5-yl)-piperazin ist bekannt aus DE 4333254.

Gegenstand der Erfindung ist ferner die Verwendung der Verbindungen der Formel I als Zwischenprodukte zur Synthese von Arzneimitteln, die Wirkungen auf das Zentralnervensystem zeigen.

Gegenstand der Erfindung ist ferner die Verwendung der Verbindungen der Formel I und/oder ihrer physiologisch unbedenklichen Salze zur Herstellung pharmazeutischer Zubereitungen, insbesondere auf nichtchemischem Wege. Hierbei können sie zusammen mit mindestens einem festen, flüssigen und/oder halbflüssigen Träger- oder Hilfsstoff und gegebenenfalls in Kombination mit einem oder mehreren weiteren Wirkstoffen in eine geeignete Dosierungsform gebracht werden.

Gegenstand der Erfindung sind ferner pharmazeutische Zubereitungen, enthaltend mindestens eine Verbindung der Formel I und/oder eines ihrer physiologisch unbedenklichen Salze.

Diese Zubereitungen können als Arzneimittel in der Human- oder Veterinärmedizin verwendet werden. Als Trägerstoffe kommen organische oder anorganische Substanzen in Frage, die sich für die enterale (z.B. orale), parenterale oder topische Applikation eignen und mit den neuen Verbindungen nicht reagieren, beispielsweise Wasser, pflanzliche Öle, Benzylalkohole, Alkylenglykole, Polyethylenglykole, Glycerintriacetat, Gelatine, Kohlehydrate wie Lactose oder Stärke, Magnesiumstearat, Talk, Vaseline. Zur oralen Anwendung dienen insbesondere Tabletten, Pillen, Dragees, Kapseln, Pulver, Granulate, Sirupe, Säfte oder Tropfen, zur rektalen Anwendung Suppositorien, zur parenteralen Anwendung Lösungen, vorzugsweise ölige oder wässrige Lösungen, ferner Suspensionen, Emulsionen oder Implantate, für die topische Anwendung Salben, Cremes oder Puder. Die neuen Verbindungen können auch lyophilisiert und die erhaltenen Lyophilisate z.B. zur Herstellung von Injektionspräparaten verwendet werden. Die angegebenen Zubereitungen können sterilisiert sein und/oder Hilfsstoffe wie Gleit-, Konservierungs-, Stabilisierungs- und/oder Netzmittel, Emulgatoren, Salze zur Beeinflussung des osmotischen Druckes, Puffersubstanzen, Farb-, Geschmacks- und /oder mehrere weitere Wirkstoffe enthalten, z.B. ein oder mehrere Vitamine.

Die Verbindungen der Formel I und ihre physiologisch unbedenklichen Salze können bei der Bekämpfung von Krankheiten verwendet werden.

Vor- und nachstehend sind alle Temperaturen in °C angegeben. In den nachfolgenden Beispielen bedeutet "übliche Aufarbeitung": Man gibt, falls erforderlich, Wasser hinzu, stellt, falls erforderlich, je nach Konstitution des Endprodukts auf pH-Werte zwischen 2 und 10 ein, extrahiert mit Ethylacetat oder Dichlormethan, trennt ab, trocknet die organische Phase über Natriumsulfat, dampft ein und reinigt durch Chromatographie an Kieselgel und /oder durch Kristallisation. Rf-Werte an Kieselgel.

### Beispiel 1

Eine Lösung von 2,3 g 5-Nitrobenzofuran-2-carbonsäureethylester in 60 ml Methanol wird in Gegenwart von Raney-Nickel hydriert. Der Katalysator wird abfiltriert und die Lösung eingeengt. Man erhält nach üblicher Aufarbeitung 5-Aminobenzofuran-2-carbonsäureethylester, Rf 0,1 (Dichlormethan/Ethanol 9,5 : 0,5); Hydrochlorid F. 246-248°.

### Beispiel 2

Eine Lösung von 2,05 g 5-Aminobenzofuran-2-carbonsäureethylester in 80 ml Dichlormethan wird mit 1,5 g N,N-Bis-(2-chlorethyl)-amin versetzt und 10 Stunden gerührt. Man arbeitet wie üblich auf und erhält 5-(1-Piperazinyl)-benzofuran-2-carbonsäureethylester, Rf 0,55(Isopropanol/Wasser 95 : 5).

### Beispiel 3

Eine Lösung von 1 g 5-(1-Piperazinyl)-benzofuran-2-carbonsäureethylester in 50 ml THF wird mit 1 g Di-tert.-butyl-dicarbonat 3 Stunden gerührt. Nach üblicher Aufarbeitung erhält man 5-(4-tert.-Butyloxycarbonyl-1-piperazinyl)-benzofuran-2-carbonsäureethylester, F. 116-118°.

### Beispiel 4

Eine Lösung von 3 g 5-(4-tert.-Butyloxycarbonyl-1-piperazinyl)-benzofuran-2-carbonsäureethylester in 100 ml N-Methylpyrrolidon wird mit 1 g Formamid und 3 g Natriumalkoholat 5 Stunden gerührt. Nach üblicher Aufarbeitung erhält man 5-(4-tert.-Butyloxycarbonyl-1-piperazinyl)-benzofuran-2-carboxamid, F. 198-200°.

### Beispiel 5

Eine Lösung von 1 g 5-(1-Piperazinyl)-benzofuran-2-carbonsäureethylester in 50 ml Dichlormethan wird mit 1 g Benzylchlorid versetzt und 2 Stunden gerührt. Nach üblicher Aufarbeitung erhält man 5-(4-Benzyl-1-piperazinyl)-benzofuran-2-carbonsäureethylester, F. 219-222°.

### Beispiel 6

1 g 5-(4-tert.-Butyloxycarbonyl-1-piperazinyl)-benzofuran-2-carboxamid wird in 50 ml methanolischer HCI gelöst und 1 Stunde gerührt. Nach üblicher Aufarbeitung erhält man 5-(1-Piperazinyl)-benzofuran-2-carboxamid, F. 252-255°.

Die nachfolgenden Beispiele betreffen pharmazeutische Zubereitungen:

### Beispiel A: Injektionsgläser

Eine Lösung von 100 g eines Wirkstoffes der Formel II und 5 g Dinatnumhydrogenphosphat wird in 3 I zweifach destilliertem Wasser mit 2 n Salzsäure auf pH 6,5 eingestellt, steril filtriert, in Injektionsgläser abgefüllt, unter sterilen Bedingungen lyophilisiert und steril verschlossen. Jedes Injektionsglas enthält 5 mg Wirkstoff.

### Beispiel B: Suppositorien

Man schmilzt ein Gemisch von 20 g eines Wirkstoffes der Formel I mit 100 g Sojalecithin und 1400 g Kakaobutter, gießt in Formen und läßt erkalten. Jedes Suppositorium enthält 20 mg Wirkstoff.

### Beispiel C: Lösung

Man bereitet eine Lösung aus 1 g eines Wirkstoffes der Formel I, 9,38 g NaH₂PO₄ · 2 H₂O, 28,48 g Na₂HPO₄ ·12 H₂O und 0,1 g Benzalkoniumchlorid in 940 ml zweifach destilliertem Wasser. Man stellt auf pH 6,8 ein, füllt auf 1 I auf und sterilisiert durch Bestrahlung. Diese Lösung kann in Form von Augentropfen verwendet werden.

### Beispiel D: Salbe

Man mischt 500 mg eines Wirkstoffes der Formel I mit 99,5 g Vaseline unter aseptischen Bedingungen.

### Beispiel E: Tabletten

Ein Gemisch von 1 kg Wirkstoff der Formel I, 4 kg Lactose, 1,2 kg Kartoffelstärke, 0,2 kg Talk und 0,1 kg Magnesiumstearat wird in üblicher Weise zu Tabletten verpreßt, derart, daß jede Tablette 10 mg Wirkstoff enthält.

### Beispiel F: Dragees

Analog Beispiel E werden Tabletten gepreßt, die anschließend in üblicher Weise mit einem Überzug aus Saccharose, Kartoffelstärke, Talk, Tragant und Farbstoff überzogen werden.

### Beispiel G: Kapseln

2 kg Wirkstoff der Formel I werden in üblicher Weise in Hartgelatinekapseln gefüllt, so daß jede Kapsel 20 mg des Wirkstoffs enthält.

### Beispiel H: Ampullen

Eine Lösung von 1 kg Wirkstoff der Formel I in 60 I zweifach destilliertem Wasser wird steril filtriert, in Ampullen abgefüllt, unter sterilen Bedingungen lyophilisiert und steril verschlossen. Jede Ampulle enthält 10 mg Wirkstoff.

## Patentansprüche

1. Benzofurane der Formel I 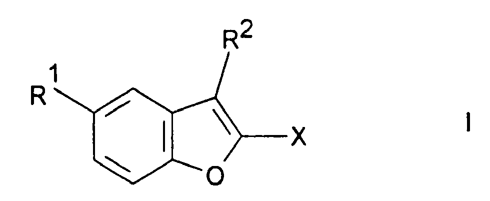 worin
R¹ NH₂ oder 1-Piperazinyl,
R² H, Cl, Br, OH oder OA,
X CONR⁴R⁵ oder CO-Het,
R⁴ und R⁵ jeweils unabhängig voneinander H, A oder Benzyl,
A Alkyl mit 1-4 C-Atomen,
Het Imidazol-1-yl, Triazol-1-yl oder Tetrazol-1-yl und
bedeuten,
sowie deren Salze.

2. Verbindungen nach Anspruch 1:
a) 5-(1-Piperazinyl)-benzofuran-2-carboxamid,
b) 5-Amino-benzofuran-2-carboxamid
sowie deren Salze.

3. Verfahren zur Herstellung von Benzofuranen der Formel I nach Anspruch 1 sowie von deren Salzen, **dadurch gekennzeichnet, daß** man eine Verbindung, die der Formel I entspricht,
worin statt R¹ eine Nitrogruppe steht,
auf übliche Weise reduziert,
oder
daß man eine Verbindung, die der Formel I entspricht,
worin R¹ eine NH₂-Gruppe ist,
mit einer Verbindung der Formel II
R⁶N(CH₂CH₂X¹)₂ II
worin
R⁶ H oder Benzyl und
X¹ Cl, Br, I, OH oder eine reaktionsfähig funktionell abgewandelte OH-Gruppe bedeutet,
umsetzt,
oder
daß man eine Verbindung, die der Formel I entspricht, worin statt X eine COOA-Gruppe steht, worin A die angegebene Bedeutung
besitzt,
in eine andere Verbindung der Formel I umwandelt, in der X CONR⁴R⁵ bedeutet, worin R⁴ und R⁵ die angegebenen Bedeutungen besitzen,
oder
daß man eine Verbindung, die der Formel I entspricht, worin statt X eine COOH-Gruppe steht,
in eine andere Verbindung der Formel I umwandelt, in der X CO-Het bedeutet, worin Het die angegebene Bedeutung besitzt,
oder
daß man eine Verbindung, die der Formel I entspricht,
worin statt R¹ eine 4-R³-Piperazinylgruppe steht, worin R³ Benzyl oder eine Aminoschutzgruppe bedeutet,
durch Abspaltung der Schutzgruppe in eine Verbindung der Formel I umwandelt, in der R¹ 1-Piperazinyl bedeutet
und/oder
daß man eine Base der Formel I durch Behandeln mit einer Säure in eines ihrer Salze umwandelt.

4. Arzneimittel der Formel I nach Anspruch 1 und ihre physiologisch unbedenklichen Salze.

5. Verwendung der Verbindungen der Formel I nach Anspruch 1 als Zwischenprodukte zur Synthese von Arzneimitteln.

6. Verwendung gemäß Anspruch 5 der Verbindungen der Formel I nach Anspruch 1 als Zwischenprodukte zur Synthese von Arzneimitteln, die Wirkungen auf das Zentralnervensystem zeigen.

7. Verwendung gemäß Anspruch 5 der Verbindungen der Formel I nach Anspruch 1 bei der Synthese von
1-[4-(5-Cyan-indol-3-yl)-butyl]-4-(2-carbamoyl-benzofuran-5-yl)-piperazin und dessen Salzen, **dadurch gekennzeichnet,**
**daß** man 2-(2-Formylphenoxy)-essigsäure nitriert,
**daß** man die so erhaltene 2-(2-Formyl-4-nitrophenoxy)-essigsäure cyclisiert,
**daß** man die so erhaltene 5-Nitrobenzofuran-2-carbonsäure verestert,
**daß** man die so erhaltene Verbindung der Formel III 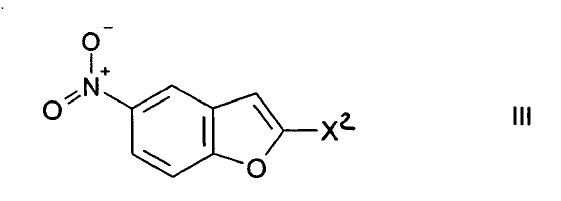 worin X² COOA, COOPh oder COOCH₂Ph
bedeutet,
reduziert,
**daß** man die so erhaltene Verbindung der Formel I,
worinR¹ NH₂ und anstelle von X eine Gruppe COOA, COOPh oder COOCH₂Ph steht,
in ihr Säureadditionssalz umwandelt und dieses mit einem Säureadditionssalz einer Verbindung der Formel II
HN(CH₂CH₂X¹)₂ II
worin X¹ Cl, Br, I, OH oder eine reaktionsfähig funktionell abgewandelte OH-Gruppe bedeutet,
zu einem Piperazinderivat der Formel IV 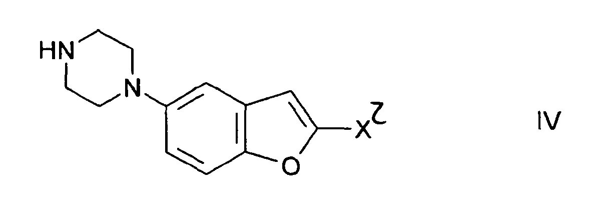 worin X² COOA, COOPh oder COOCH₂Ph bedeutet
umsetzt,
**daß** man die so erhaltene Verbindung der Formel IV
in ihr Säureadditionssalz umwandelt,
**daß** man dieses mit einer Verbindung umsetzt, die zur Einführung einer Aminoschutzgruppe geeignet ist,
**daß** man die so erhaltene Verbindung der Formel V 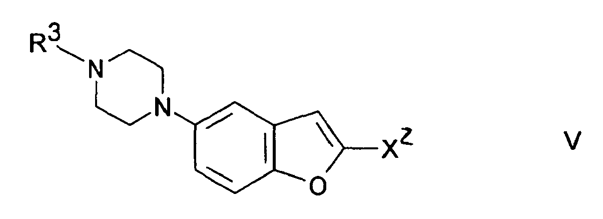 worin R³ Aminoschutzgruppe bedeutet und X² die angegebene Bedeutung besitzt,
in eine Verbindung der Formel VI 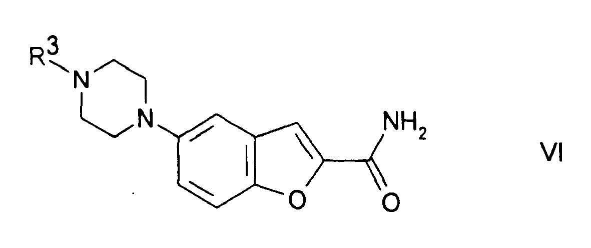 worin R³ die obige Bedeutung hat,
umwandelt,
**daß** man die so erhaltene Verbindung der Formel VI durch Abspaltung der Aminoschutzgruppe in 5-(1-Piperazinyl)-benzofuran-2-carboxamid oder ein Säureadditionssalz umwandelt, und
**daß** man 5-(1-Piperazinyl)-benzofuran-2-carboxamid mit 3-(4-Chlorbutyl)-5-cyan-indol zu 1-[4-(5-Cyan-indol-3-yl)-butyl]-4-(2-carbamoyl-benzofuran-5-yl)-piperazin umsetzt und gegebenenfalls in sein Säureadditionssalz überführt.

8. Verfahren zur Herstellung pharmazeutischer Zubereitungen, **dadurch gekennzeichnet, daß** man eine Verbindung der Formel I nach Anspruch 1 und/oder eines ihrer physiologischen unbedenklichen Salze zusammen mit mindestens einem festen, flüssigen oder halbflüssigen Träger- oder Hilfsstoff in eine geeignete Dosierungsform bringt.

9. Pharmazeutische Zubereitung, **gekennzeichnet durch** einen Gehalt an mindestens einer Verbindung der Formel I nach Anspruch 1 und/oder einem ihrer physiologisch unbedenklichen Salze.

10. Verwendung von Verbindungen der Formel I nach Anspruch 1 und ihre physiologisch unbedenklichen Salze zur Herstellung einer pharmazeutischen Zubereitung zur Bekämpfung von Krankheiten.

## Claims

1. Benzofurans of the formula I 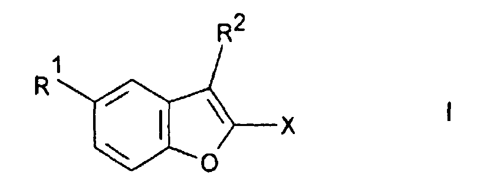 in which
R¹ denotes NH₂ or 1-piperazinyl,
R² denotes H, Cl, Br, OH or OA,
X denotes CONR⁴R⁵ or CO-Het,
R⁴ and R⁵ each, independently of one another, denote H, A or benzyl,
A denotes alkyl having 1-4 C atoms,
Het denotes imidazol-1-yl, triazol-1-yl or tetrazol-1-yl,
and salts thereof.

2. Compounds according to Claim 1:
a) 5-(1-piperazinyl)benzofuran-2-carboxamide;
b) 5-aminobenzofuran-2-carboxamide;
and salts thereof

3. Process for the preparation of benzofurans of the formula I according to Claim 1 and salts thereof, **characterised in that**
a compound which conforms to the formula I
in which a nitro group is present instead of R¹
is reduced in a conventional manner,
or
**in that** a compound which conforms to the formula I
in which R¹ is an NH₂ group
is reacted with a compound of the formula II
R⁶N(CH₂CH₂X¹)₂ II
in which
R⁶ denotes H or benzyl, and
X¹ denotes Cl, Br, I, OH or a reactively functionally modified OH group,
or
**in that** a compound which conforms to the formula I in which a COOA group, in which A has the meaning indicated, is present instead of X is converted into another compound of the formula I in which X denotes CONR⁴R⁵, in which R⁴ and R⁵ have the meanings indicated, or
**in that** a compound which conforms to the formula I in which a COOH group is present instead of X
is converted into another compound of the formula I in which X denotes CO-Het, in which Het has the meaning indicated,
or
**in that** a compound which conforms to the formula I
in which a 4-R³-piperazinyl group, in which R³ denotes benzyl or an amino-protecting group, is present instead of R¹
is converted into a compound of the formula I in which R¹ denotes 1-piperazinyl by removal of the protecting group,
and/or
**in that** a base of the formula I is converted into one of its salts by treatment with an acid.

4. Medicament of the formula I according to Claim 1 and physiologically acceptable salts thereof.

5. Use of the compounds of the formula I according to Claim 1 as intermediates for the synthesis of medicaments.

6. Use according to Claim 5 of the compounds of the formula I according to Claim 1 as intermediates for the synthesis of medicaments which exhibit actions on the central nervous system.

7. Use according to Claim 5 of the compounds of the formula I according to Claim 1 in the synthesis of
1-[4-(5-cyanoindol-3-yl)butyl]-4-(2-carbamoylbenzofuran-5-yl)piperazine and salts thereof, **characterised in that**
2-(2-formylphenoxy)acetic acid is nitrated,
**in that** the 2-(2-formyl-4-nitrophenoxy)acetic acid obtained in this way is cyclised,
**in that** the 5-nitrobenzofuran-2-carboxylic acid obtained in this way is esterified,
**in that** the compound of the formula III 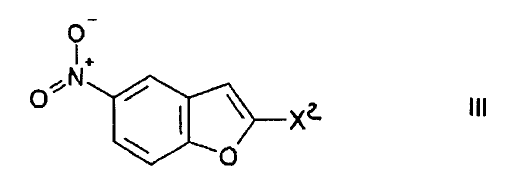 in which X² denotes COOA, COOPh or COOCH₂Ph,
obtained in this way is reduced,
**in that** the compound of the formula I,
in which R¹ denotes NH₂ and a COOA, COOPh or COOCH₂Ph group is present instead of X,
obtained in this way is converted into its acid-addition salt, and this is reacted with an acid-addition salt of a compound of the formula II
HN(CH₂CH₂X¹)₂ II
in which X¹ denotes Cl, Br, I, OH or a reactively functionally modified OH group
to give a piperazine derivative of the formula IV 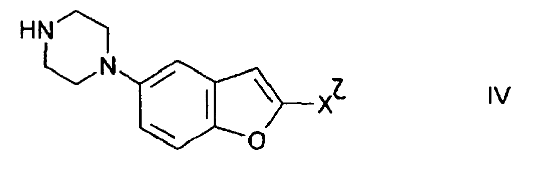 in which X² denotes COOA, COOPh or COOCH₂Ph,
**in that** the compound of the formula IV obtained in this way
is converted into its acid-addition salt,
**in that** this is reacted with a compound which is suitable for the introduction of an amino-protecting group,
**in that** the compound of the formula V 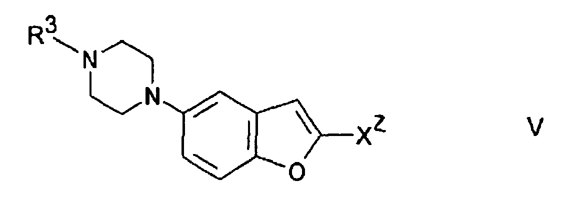 in which R³ denotes an amino-protecting group and X² has the meaning indicated,
obtained in this way is converted into a compound of the formula VI 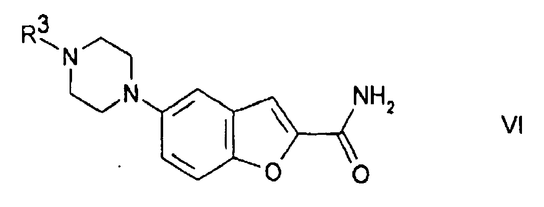 in which R³ has the above meaning,
**in that** the compound of the formula VI obtained in this way is converted into 5-(1-piperazinyl)benzofuran-2-carboxamide or an acid-addition salt by removal of the amino-protecting group, and
**in that** 5-(1-piperazinyl)benzofuran-2-carboxamide is reacted with 3-(4-chlorobutyl)-5-cyanoindole to give 1-[4-(5-cyanoindol-3-yl)butyl]-4-(2-carbamoylbenzofuran-5-yl)piperazine, and
if desired converted into its acid-addition salt.

8. Process for the preparation of pharmaceutical compositions, **characterised in that** a compound of the formula I according to Claim 1 and/or one of its physiologically acceptable salts is converted into a suitable dosage form together with at least one solid, liquid or semiliquid excipient or adjuvant.

9. Pharmaceutical composition, **characterised by** a content of at least one compound of the formula I according to Claim 1 and/or one of its physiologically acceptable salts.

10. Use of compounds of the formula I according to Claim 1 and physiologically acceptable salts thereof for the preparation of a pharmaceutical composition for combating diseases.

## Revendications

1. Benzofuranes de la formule I 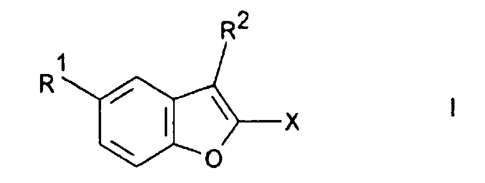 dans laquelle
R¹ représente NH₂ ou 1-pipérazinyle,
R² représente H, CI, Br, OH ou OA,
X représente CONR⁴R⁵ ou CO-Het,
R⁴ et R⁵ chacun indépendamment de l'autre, représentent H, A ou benzyle,
A représente alkyle comportant de 1 à 4 atomes de C,
Het représente imidazol-1-yle, triazol-1-yle ou tétrazol-1-yle,
et leurs sels.

2. Composés selon la revendication 1 :
a) 5-(1-pipérazinyl)benzofurane-2-carboxamide ;
b) 5-aminobenzofurane-2-carboxamide ;
et leurs sels.

3. Procédé pour la préparation de benzofuranes de la formule 1 selon la revendication 1 et de leurs sels, **caractérisé en ce qu'**un composé qui est conforme à la formule I où un groupe nitro est présent au lieu de R¹ est réduit d'une manière classique,
ou
**en ce qu'**un composé qui est conforme à la formule I où R¹ est un groupe NH₂ est amené à réagir avec un composé de la formule II
R⁶N(CH₂CH₂X¹)₂ II
dans laquelle
R⁶ représente H ou benzyle, et
X¹ représente CI, Br, I, OH ou un groupe OH modifié fonctionnellement réactivement,
ou
**en ce qu'**un composé qui est conforme à la formule I où un groupe COOA, où A présente la signification indiquée, est présent au lieu de X est converti selon un autre composé de la formule I où X représente CONR⁴R⁵, où R⁴ et R⁵ présentent les significations indiquées,
ou
**en ce qu'**un composé qui est conforme à la formule I où un groupe COOH est présent au lieu de X est converti selon un autre composé de la formule I où X représente CO-Het, où Het présente la signification indiquée,
ou
**en ce qu'**un composé qui est conforme à la formule I, où un groupe 4-R³-pipérazinyle, où R³ représente benzyle ou un groupe de protection d'amino, est présent au lieu de R¹, est converti selon un composé de la formule I où R¹ représente 1-pipérazinyle par enlèvement du groupe de protection,
et/ou
**en ce qu'**une base de la formule I est convertie selon l'un de ses sels par traitement avec un acide.

4. Médicament de la formule I selon la revendication 1 et ses sels physiologiquement acceptables.

5. Utilisation des composés de la formule I selon la revendication 1 en tant que produits intermédiaires pour la synthèse de médicaments.

6. Utilisation selon la revendication 5 des composés de la formule I selon la revendication 1 en tant que produits intermédiaires pour la synthèse de médicaments qui présentent des actions sur le système nerveux central.

7. Utilisation selon la revendication 5 des composés de la formule I selon la revendication 1 au niveau de la synthèse de
1-[4-(5-cyanoindol-3-yl)butyl]-4-(2-carbamoylbenzofurane-5-yl)-pipérazine et de ses sels, **caractérisée en ce que** de l'acide 2-(2-formylphénoxy)acétique est nitré,
**en ce que** l'acide 2-(2-formyl-4-nitrophénoxy)acétique obtenu de cette façon est cyclisé,
**en ce que** l'acide 5-nitrobenzofurane-2-carboxylique obtenu de cette façon est estérifié,
**en ce que** le composé de la formule III 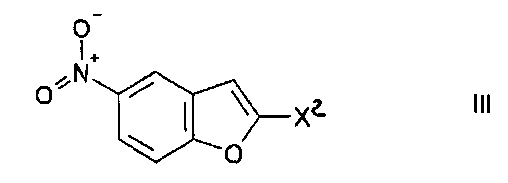 dans laquelle X² représente COOA, COOPh ou COOCH₂Ph,
obtenu de cette façon est réduit,
**en ce que** le composé de la formule I,
dans laquelle R¹ représente NH₂ et un groupe COOA, COOPh ou COOCH₂Ph est présent au lieu de X,
obtenu de cette façon est converti selon son sel par addition d'acide, et celui-ci est amené à réagir avec un sel par addition d'acide d'un composé de la formule II
HN(CH₂CH₂X¹)₂ II
dans laquelle X¹ représente CI, Br, I, OH ou un groupe OH modifié fonctionnellement réactivement
pour obtenir un dérivé de pipérazine de la formule IV 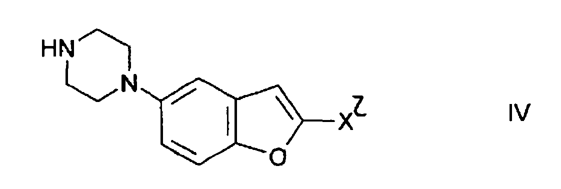 dans laquelle X² représente COOA, COOPh ou COOCH₂Ph,
**en ce que** le composé de la formule IV obtenu de cette façon est converti selon son sel par addition d'acide,
**en ce que** celui-ci est amené à réagir avec un composé qui convient pour l'introduction d'un groupe de protection d'amino,
**en ce que** le composé de la formule V 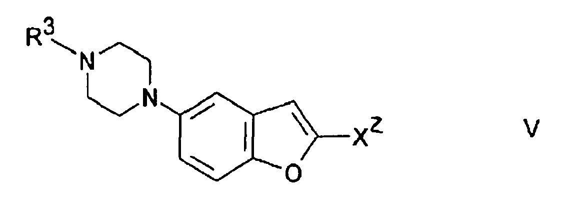 dans laquelle R³ représente un groupe de protection d'amino et X² présente la signification indiquée,
obtenu de cette façon est converti selon un composé de la formule VI 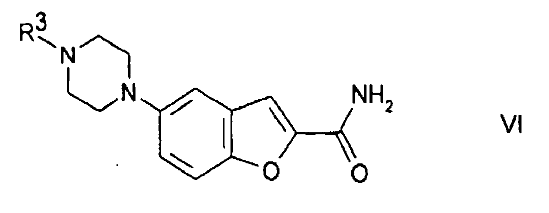 dans laquelle R³ présente la signification mentionnée ci-avant,
**en ce que** le composé de la formule VI obtenu de cette façon est converti selon 5-(1-pipérazinyl)benzofurane-2-carboxamide ou un sel par addition d'acide par enlèvement du groupe de protection d'amino, et
**en ce que** 5-(1-pipérazinyl)benzofurane-2-carboxamide est amené à réagir avec 3-(4-chlorobutyl)-5-cyanoindole pour obtenir 1-[4-(5-cyanoindol-3-yl)butyl]-4-(2-carbamoylbenzofurane-5-yl)pipérazine, et
si souhaité, est converti selon son sel par addition d'acide.

8. Procédé pour la préparation de compositions pharmaceutiques, **caractérisé en ce qu'**un composé de la formule I selon la revendication 1 et/ou l'un de ses sels physiologiquement acceptables est converti selon une forme de dosage appropriée en association avec au moins un excipient ou adjuvant solide, liquide ou semi-liquide.

9. Composition pharmaceutique, **caractérisée par** une teneur en au moins un composé de la formule I selon la revendication 1 et/ou l'un de ses sels physiologiquement acceptables.

10. Utilisation de composés de la formule I selon la revendication 1 et de ses sels physiologiquement acceptables pour la préparation d'une composition pharmaceutique pour combattre des maladies.
